# EUROPEAN PATENT APPLICATION

(11) **EP 4 289 812 A1**
(43) Date of publication of application: **13.12.2023**
(21) Application number: 22926328.0
(22) Date of filing: 15.09.2022
(51) Int. Cl.: C07C 51/373, C07C 59/19, C07C 51/15, C07C 51/42, C07C 51/487, C07C 51/48, C01B 32/50

(54) **METHOD FOR SYNTHESIZING CARBON ISOTOPE-LABELED PYRUVIC ACID**

(30) Priority: 13.04.2022 CN 202221036401 U; 13.04.2022 CN 202210387770
(71) Applicant: Shenzhen Zhonghe Headway Bio-Sci & Tech Co., Ltd., Shenzhen, Guangdong 518057 (CN)
(72) Inventor: The designation of the inventor has not yet been filed
(74) Representative: Vitina, Maruta
(86) International application number: PCT/CN2022/118953
(87) International publication number: WO 2023/197522

(57) **Abstract**

A method for synthesizing a labeled pyruvic acid is provided. An isopropenyl Grignard reagent is added to a reaction vessel and cooled to a preset temperature. A carbon isotope-labeled carbon dioxide is added to the reaction vessel and continuously reacted with the isopropenyl Grignard reagent for a preset time. The reaction system is added with an acid and adjusted to a preset temperature to quench the reaction. The reaction system is subjected to ozonization, and reduced in the presence of a reducing agent. Impurities are removed to obtain the labeled pyruvic acid.

## Description

### TECHNICAL FIELD

This application relates to synthesis of isotope-labeled drugs, and more particularly to a method for synthesizing a labeled pyruvic acid.

### BACKGROUND

Magnetic Resonance Imaging (MRI) is able to obtain global or local images of a patient's body in a non-invasive manner, which improves the safety of medical examinations by avoiding exposing the patient and medical personnel to potentially harmful radiation. The high-quality MRI images rely on the use of MR contrast agents, which are predominated by ¹³C MR contrast agents. Considering that pyruvic acid is a key intermediate metabolite in cellular metabolism, the [1-¹³C]pyruvic acid has become the predominant hyperpolarized (HP) bio-probe. Similar to[1-¹³C] pyruvic acid, the [1-¹⁴C]pyruvic acid is a radiolabeled compound, and can be used as a tracer for pharmacokinetic experiments. [1-¹³C]pyruvic acid and [1-¹⁴C]pyruvic acid are prepared by similar methods and processes., and the main [1-¹³C]pyruvic acid synthesis strategies are listed as follows.

International patent publication No. 2008/011033 A2 discloses a synthetic method of isotopically labeled α-keto acids and esters, in which a 2-propenylmagnesium bromide Grignard reagent is produced from 2-bromopropene, and subsequently undergoes an addition reaction with ¹³CO₂ at low temperatures to obtain [1-¹³C]methacrylic acid, which is subjected to benzyl protection, ozonization and hydrogenation to remove the benzyl group to obtain the [1-¹³C]pyruvic acid, as shown in the following synthesis route:

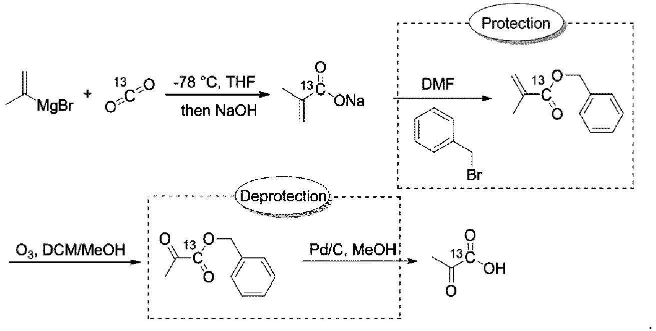

U.S. Patent Publication No. 2006/178534A discloses another synthetic route of pyruvic acid in which 1-¹³C methyl phenyl sulfoxide is used as the starting material to yield an alkyl lithium reagent, which subsequently reacts with ethyl acetate to give 1-¹³C-phenylmethylsulfoxide-acetone. The 1-¹³C-phenylmethylsulfoxide-acetone is subjected to dichloro-substitution and deprotection to produce [1-¹³C]pyruvic acid, as shown in the following synthesis route:

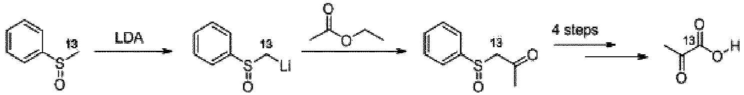

As disclosed by U.S. patent publication No. 2006/178534A, the alkyl lithium reagent is prepared by using methyl phenyl sulfide as the starting material, and reacts with ¹³CO₂ to obtain 1-phenylthio-[1-¹³C]-acetic acid, which then undergoes chlorination, amidation, chlorination and ethanol substitution to obtain ethyl [1-¹³C]-N, N-dimethyl oxalate. The ethyl [1-¹³C]-N, N-dimethyl oxalate further reacts with the alkyl lithium reagent to obtain a ketone compound, which undergoes reduction, removal of phenylsulfinyl, and hydrochloric acid hydrolysis to obtain the target product [l-¹³C]pyruvic acid.

Chinese patent application publication No. 109096092A discloses that acetaldehyde undergoes condensation with dithiol to obtain a thioacetal intermediate, which reacts with n-butyllithium to yield the corresponding alkyl lithium reagent. Then the thioacetal alkyl lithium reagent undergoes an addition reaction with ¹³CO₂ to obtain ¹³C-labeled dithioketal, and finally the [1-¹³C]pyruvic acid could be obtained by removing the dithiol protecting group, as shown in the following synthetic route:

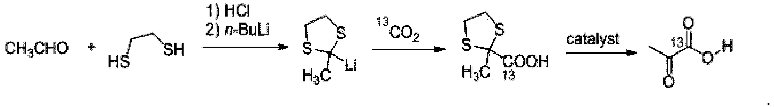

The above synthesis methods of [1-¹³C]pyruvic acid all suffer from cumbersome operation, poor synthesis efficiency, complex raw materials, and high cost. Moreover, these methods all require multiple steps for separation and purification, resulting in high operational difficulty. Such defects also exist in the preparation of [1-¹⁴C]pyruvic acid.

### SUMMARY

In view of the above deficiencies in the prior art, this application provides a method for synthesizing a labeled pyruvic acid with fewer steps, high synthesis efficiency, low cost, and simple product extraction.

This application provides a method for synthesizing a labeled pyruvic acid, comprising:
(S1) adding an isopropenyl Grignard regent to the reaction vessel followed by cooling to a preset temperature;
(S2) adding a carbon isotope-labeled carbon dioxide to the reaction vessel followed by reaction with the isopropenyl Grignard reagent for a preset time;
(S3) adding an acid to a reaction system obtained in step (S2) and adjusting the reaction system to a preset temperature to quench the reaction;
(S4) subjecting a quenched reaction system to ozonization to obtain an ozonized product;
(S5) reducing the ozonized product obtained in step (S4) with a reducing agent to obtain a reduced product; and
(S6) removing impurities from the reduced product to obtain the labeled pyruvic acid.

In one embodiment, in step (S4), ozone is introduced to the quenched reaction system to continuously perform the ozonization for a preset time.

In one embodiment, step (S4) includes:
(S41) subjecting the quenched reaction system to extraction, and combing the organic phases to obtain an oily product; and
(S42) adding a solvent to the oily product followed by cooling to a preset temperature and introduction of ozone to carry out the ozonization for a preset time.

In one embodiment, in step (S3), the reaction system is added with a dilute hydrochloric acid to reach a pH of 3 or less.

In one embodiment, before step (S42), the step (S4) further includes:
(S411) transferring the oil product to another reaction vessel;
wherein in step (42), the solvent is a mixture of dichloromethane and methanol.

In one embodiment, the carbon isotope-labeled carbon dioxide is ¹³C-labeled carbon dioxide or ¹⁴C-labeled carbon dioxide.

In one embodiment, in step (S1), before the isopropenyl Grignard reagent is added to the reaction vessel, a magnetic stirring bar is added to the reaction vessel, and argon is introduced to the reaction vessel; and the cooling is performed in an ethanol bath using a cryogenic recirculation pump.

In one embodiment, the isopropenyl Grignard reagent is cooled to -30 to -78°C, and then reacted with the carbon isotope-labeled carbon dioxide for 1h; and in step (S3), the preset temperature is -30 to 0°C.

In one embodiment, the reducing agent in step (S5) is dimethyl sulfide or triphenylphosphine.

In one embodiment, in step (S6), removal of impurities from the reduced product includes:
(S61) removing an organic phase of the reduced product;
(S62) adjusting the reduced product to be alkaline followed by extraction and separation, and combining aqueous phases;
(S63) adjusting a combined aqueous phase to be acidic followed by extraction, and combining organic phases; and
(S64) subjecting a combined organic phase obtained in step (S63) to drying, filtration and concentration to obtain the labeled pyruvic acid.

The method of synthesizing labeled pyruvic acid of this application uses an isopropenyl Grignard reagent and a carbon isotope-labeled carbon dioxide as the starting materials, and there is no need to carry out the "carboxyl protection" and "deprotection" operations. Compared with the traditional four-step method for synthesizing labeled pyruvic acid, this application can synthesize the labeled pyruvic acid in only two steps, which can simplify synthesis process, increase the synthesis efficiency, and reduce the consumption of reagents, allowing for lowered synthesis cost. Moreover, the synthetic reaction will not introduce any other reagents, and thus it is only needed to extract the labeled pyruvic acid, thereby reducing the extraction difficulty.

### BRIEF DESCRIPTION OF THE DRAWINGS

Fig. 1 is a flow chart of a method for synthesizing a labeled pyruvic acid in accordance with one embodiment of the present disclosure;
Fig. 2 is a flow chart of a one-pot synthesis method of a labeled pyruvic acid in accordance with one embodiment of the present disclosure;
Fig. 3 is a flow chart of a two-step synthesis method of a labeled pyruvic acid in accordance with one embodiment of the present disclosure;
Fig. 4 is a HPLC (high performance liquid chromatography) chromatogram of a labeled pyruvic acid in accordance with one embodiment of the present disclosure;
Fig. 5 is a mass spectrum (MS) of the labeled pyruvic acid in accordance with one embodiment of the present disclosure; and
Fig. 6 is a H-nuclear magnetic resonance (NMR) spectrum of the labeled pyruvic acid in accordance with one embodiment of the present disclosure.

### DETAILED DESCRIPTION OF EMBODIMENTS

This application will be described in detail below with reference to the accompanying drawings and embodiments to make objects, technical features and advantages of this application clearer and better understood. Many specific details are set forth in the following description to facilitate the full understanding of the present disclosure. The present disclosure can be implemented in many other ways than those described herein, and those skilled in the art may make similar improvements without departing from the spirit of the present disclosure. Therefore, the present disclosure is not limited to the specific embodiments disclosed below.

Improvements are made herein on the basis of the international patent publication No. 2008011033 A2 ("prior art") to solve the problems of excessive reaction steps, large consumption of a wide variety of reagents, and poor synthesis efficiency in the existing synthesis of labeled pyruvic acids. Specifically, the prior art takes ¹³CO₂ as the labelling agent, and adopts the four-step strategy of "oxidative addition----protection of carboxyl group----ozonization----deprotection" to synthesize the [l-¹³C]pyruvic acid; while in this application (with [l-¹³C]pyruvic acid as an example), the "carboxyl group protection" and "deprotection" are eliminated, and the simplified synthesis route is shown as follows:

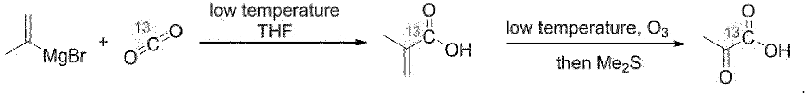

That is to say, this application can synthesize the [1-¹³C]pyruvic acid by a two-step reaction, shortening the synthesis route, reducing the types of relevant reagents and reactants, and lowering the synthesis difficulty. Similar technical effect can also be reached in the preparation of [1-¹⁴C]pyruvic acid by using the above method.

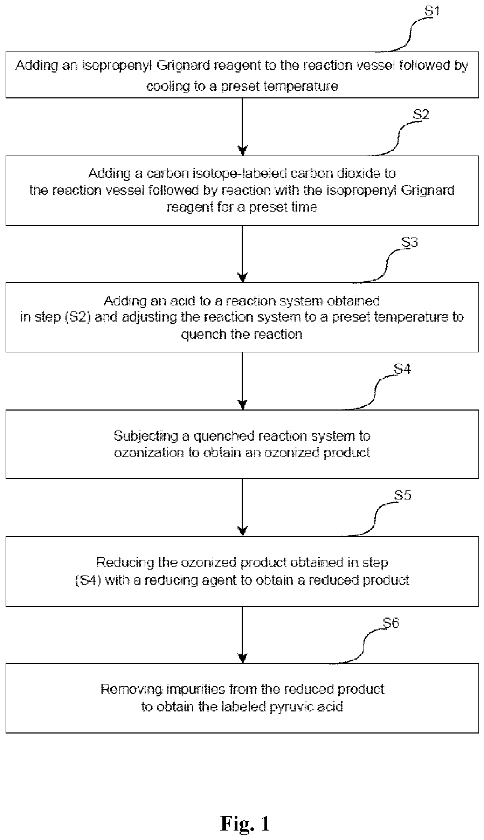

Specifically, referring to Fig. 1, the present disclosure discloses a "one-pot" method for synthesizing a labeled pyruvic acid with fewer steps, high synthetic efficiency, low cost, and simple product extraction, where the structural formula of the labeled pyruvic acid is shown as:

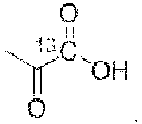

The structure of [1-¹⁴C]pyruvic acid is substantially identical to that of the [1-¹³C]pyruvic acid, with only the ¹³C replaced with ¹⁴C. The [1-¹³C]pyruvic acid is a stable isotope labeled pyruvic acid, and can be used as an MR contrast agent in the magnetic resonance imaging (MRI) to obtain a global or local image of the patient's body, facilitating the diagnosis of the patient's condition and pathological analysis.

The method provided herein for synthesizing a labeled pyruvic acid includes the following steps.

(S1) An isopropenyl Grignard reagent is added to a reaction vessel and cooled to a preset temperature.

The isopropenyl Grignard reagent is an organomentallic compound containing magnesium halide, which is a nucleophile reagent due to the presence of carbanions, and is mainly used in the organic synthesis. In addition, the Grignard reagent is a covalent compound, in which the magnesium atom is directly linked to carbon to form a polar covalent bond, and the carbon is the negatively charged end, so the Grignard reagent is an extremely strong Lewis base, capable of seizing protons from water and other Lewis acids. Therefore, during the reaction of the isopropenyl Grignard reagent with a carbon isotope-labeled carbon dioxide, it is necessary to avoid the isopropenyl Grignard reagent from being exposed to water and air. Generally, the reaction vessel is added with a magnetic stirring bar and fed with an inert gas before the isopropenyl Grignard reagent is added. Preferably, the inert gas is argon. In this embodiment, the reaction vessel is cooled down in an ethanol bath using a cryogenic circulation pump.

The magnetic stirring bar is used to mixing the materials uniformly, and the inert gas is used to replace the air in the reaction vessel to avoid the participation of oxygen and to prevent the generation of by-products, so as to reduce the collection difficulty of the target product. In the specific synthesis process, other inert gases, such as helium and nitrogen, may also be used. In addition, in this embodiment, the reaction vessel may be a single-necked round-bottomed flask or a beaker, and the cooling of the isopropenyl Grignard reagent is to provide a low-temperature environment for the reaction of the isopropenyl Grignard reagent with carbon dioxide.

(S2) The carbon isotope-labeled carbon dioxide is added proportionally to the reaction vessel and continuously reacted for a predetermined time. Preferably, the isopropenyl Grignard reagent is cooled to -30 to -78°C and then continuously reacted with the carbon isotope-labeled carbon dioxide for 1 h. In one embodiment, the carbon isotope-labeled carbon dioxide is ¹³C-labeled carbon dioxide or ¹⁴C-labeled carbon dioxide. The mechanism of the reaction of ¹³C-labeled carbon dioxide with the isopropenyl Grignard reagent for the preparation of [1-¹³C]pyruvic acid is the same as that of the reaction of ¹⁴C-labeled carbon dioxide with the isopropenyl Grignard reagent for the preparation of [1-¹⁴C]pyruvic acid, and this embodiment exemplarily illustrates the preparation process of the labeled pyruvic acid using ¹³C-labeled carbon dioxide as the reaction material.

(S3) The reaction system is added with an acid and adjusted to a preset temperature for quenching the reaction. Preferably, the reaction system is heated to -30 to 0°C for quenching the reaction.

In step (S3), the acid is hydrochloric acid. In this embodiment, hydrochloric acid is used to provide an acidic environment for the quenching reaction, and other acids, such as acetic acid and sulfuric acid, may also be applicable.

In this embodiment, the reaction mechanism of ¹³C-labeled carbon dioxide with the isopropenyl Grignard reagent is illustrated as follows:

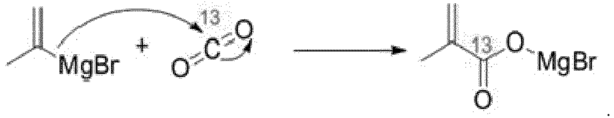

It can be seen from the reaction mechanism diagram that 2-propenylmagnesium bromide (i.e., the isopropenyl Grignard reagent) acts as a nucleophile reagent to attack the electrophilic reagent ¹³CO₂ (¹³C-labeled carbon dioxide), thereby constructing the carboxyl group to obtain a carboxyl-magnesium salt complex. The complex is quenched under the action of acid to obtain a free carboxyl group.

(S4) The reaction system is subjected to ozonization reaction after the quenching reaction. Prior to step (S5), the method further includes: introducing oxygen into the system after the ozonization reaction to drain excess ozone. Specifically, an oxygen cylinder is connected to an ozone generator, and ozone is slowly introduced into the reaction vessel below the liquid level, and after reacted for 15 min, the reaction liquid changes from light yellow to blue (namely, there is ozone dissolved in the system, indicating that the reaction is complete, and this embodiment determines the progress of the ozonization reaction based on the color of the reaction liquid). Subsequently, the ozone generator is turned off, and the oxygen is continually introduced into the reaction vessel for 5 min to drain the excess ozone.

In this embodiment, the ozonization reaction mainly includes two steps:

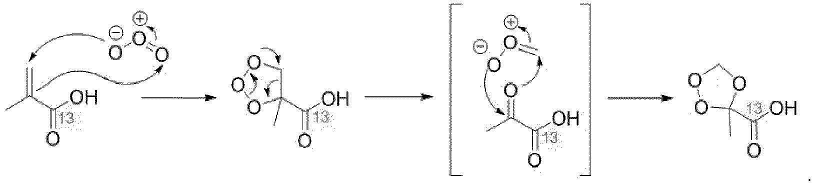

The first step of the ozonization reaction is that the C=C bond undergoes a 1, 3-dipolar cycloaddition reaction with ozone to form a primary ozonized product 1, 2, 3-trioxolane. Since the primary ozonized product is very unstable, it will be rearranged to form the relatively stable secondary ozonized product 1, 2, 4-trioxolane, which means that the final product of the ozonation reaction is the secondary ozonized product.

(S5) The product obtained in step (S4) is reduced with a reducing agent, where the reducing agent is dimethyl sulfide or triphenylphoshphine.

In the presence of dimethyl sulfide or triphenylphosphine as the reducing agent, the secondary ozonized product is decomposed to form [l-¹³C]pyruvic acid, formaldehyde, and dimethyl sulfoxide, and the reaction mechanism is as follows:

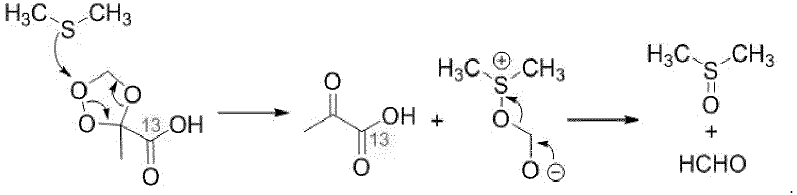

(S6) Impurities in the reduced product are removed to obtain a labeled pyruvic acid.

In the step (S6), the removal of impurities in the reduction product includes:
(S61) removing the organic phase of the reaction system;
(S62) adjusting the reaction system to be alkaline followed by extraction and separation, and combining the aqueous phases;
(S63) adjusting the combined aqueous phase to be acidic followed by extraction, and combing the organic phases; and
(S64) drying the combined organic phase in step (S3) followed by filtering and concentrating to obtain a labeled pyruvic acid, and in this embodiment, the labeled pyruvic acid is ¹³C-pyruvic acid.

The preparation of a ¹⁴C-labeled pyruvic acid further includes preparation of ¹⁴C-labeled carbon dioxide before step (S 1), which includes:
(S01) adding a predetermined weight of Ba¹⁴CO₃ to a reaction vessel;
(S02) adding concentrated sulfuric acid dropwise to the reaction vessel to allow a reaction with Ba¹⁴CO₃ to prepare the ¹⁴C-labeled carbon dioxide; and
(S03) proceeding to step (S2).

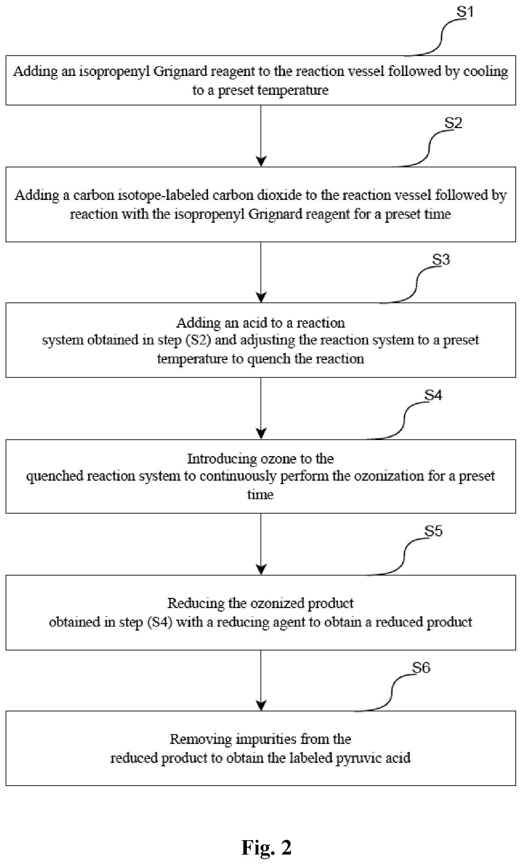

It is to be noted that, referring to an embodiment shown in Fig. 2, in step (S4), ozone is introduced to the quenched system in the reaction vessel, and the ozonization reaction is continued for a predetermined time, that is to say, in this embodiment, the two steps are always carried out in the same reaction vessel, and in this way, it is only required to ensure that the same reaction solvent is selected in both steps, and there is no need to separate the products, and the reaction mixture generated in the first pot reaction can be directly used as the raw material for the second step reaction to synthesize ¹³C pyruvic acid, as shown in the following "one-pot" synthetic route:

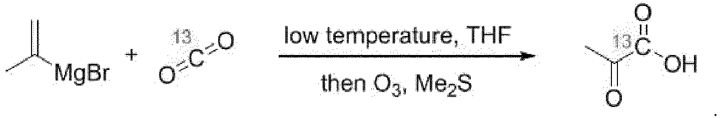

After the synthesis route is improved, all the reaction reagents and materials are introduced into the same reaction vessel in a certain order, and there is no need to separate and purify the intermediate products, that is, the ¹³C pyruvic acid can be obtained by the "one-pot" method with fewer reaction steps.

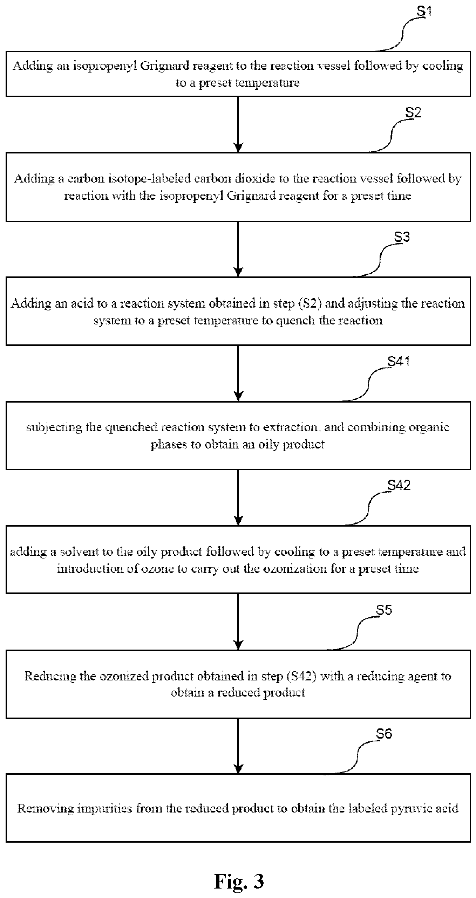

Referring to an embodiment shown in Fig. 3, step (S4) includes:
(S41) subjecting the reaction product of step (S3) to extraction, and combining the organic phases to obtain an oily product; and
(S42) adding a solvent to the oily product; cooling the oily product to -30 to -78°C followed by ozonization for a preset time.

In this embodiment, no purification is required for the product of the first step, and it is only required to perform extraction on the final product of the second step to obtain ¹³C pyruvic acid. Before step S42, it further includes:
(S411) transferring the oily product to another reaction vessel, and adding a solvent to the reaction vessel, which is a mixture of dichloromethane and methanol. In other words, in this embodiment, the synthesis of the labeled pyruvic acid is carried out separately in two reaction vessels, that is, the ¹³C pyruvic acid can be obtained by the "two-step" reaction in this embodiment.

Further, in this embodiment, in step (S3), before the quenching reaction, it also includes adjusting the reaction system to pH 3 or less with a dilute hydrochloric acid solution.

The "one-pot" preparation and the "two-step" preparation of the labeled pyruvic acid marker will be described respectively with reference to examples.

### "One-pot" preparation of labeled pyruvic acid

A magnetic stirring bar was added to a well-dried, single-necked round-bottomed 100-mL flask, which was then filled with argon gas. The round-bottomed flask was slowly added with 25 mL of an isopropenyl Grignard reagent at a concentration of 1.0 mol/L (25 mmol in total), and cooled to -78°C in an ethanol bath using a cryogenic recirculation pump, to allow for a rapid cooling of the isopropenyl Grignard reagent to the preset temperature.

A ¹³C-labeled carbon dioxide (560 mL, 25 mmol) was slowly introduced to a gas flow meter at a flow rate of 100 mL/min into the condensed reaction vessel below the liquid level. After all the ¹³C-labeled carbon dioxide gas was introduced into the reaction vessel, the reaction system was continued to be stirred at -78°C for 1 h to allow the ¹³C-labeled carbon dioxide to be fully mixed and reacted with the isopropenyl Grignard reagent. Subsequently, 3.0 mL of hydrochloric acid at a concentration of 12 N (12 mol/L) was added to the reaction system, followed by raising the temperature of the reaction vessel to -50°C.

In this embodiment, after cooled to -78°C, the isopropenyl Grignard reagent was continuously reacted with the carbon isotope-labeled carbon dioxide (¹³C-labeled carbon dioxide in this embodiment) for 1h, then an acid was added into the reaction system and heated to -50°C to carry out a quenching reaction, and the temperature rise was conducive to accelerating the quenching reaction.

In another embodiment, the isopropenyl Grignard reagent was cooled to -60°C and introduced into the ¹³C-labeled carbon dioxide for a continuous reaction for 1h. After the isopropenyl Grignard reagent was reacted with the ¹³C-labeled carbon dioxide, the temperature was lowered to -30°C for a quenching reaction to free the carboxyl group in the carboxylate-magnesium salt complex.

In an embodiment, after cooled to -50°C, the isopropenyl Grignard reagent was continuously reacted with ¹³C-labeled carbon dioxide for 1h and the acid is added into the reaction system and then heated to -20°C for the quenching reaction to further accelerate the quenching reaction.

In an embodiment, the isopropenyl Grignard reagent was cooled to -30°C and then continuously reacted with ¹³C-labeled carbon dioxide for 1 h, and the reaction system was added with the acid and then heated to -10°C to further accelerate the quenching reaction. Of course, the quenching can also be carried out at 0°C.

At the end of the ozonization, 2.2 mL of dimethyl sulfide (30 mmol) was added to the reaction vessel and subsequently raised to room temperature and stirred for 2 h to allow the product in step S4 to be fully reduced by the dimethyl sulfide.

The removal of impurities in the reduced product included: removing the organic phase of the reaction solution by rotary evaporator, followed by dissolving with 100 mL of dichloromethane, then adjusting the solution pH value to 9-11 with 10% aqueous sodium hydroxide solution, extracting and splitting the liquid, then washing the organic phase twice with deionized water, combining the aqueous phase, then adjusting the pH value to 1-5 with aqueous hydrochloric acid, then extracting the aqueous phase three times with dichloromethane, combining the organic phases, and finally drying the organic phase with anhydrous sodium sulfate. Finally, the organic phase was dried with anhydrous sodium sulfate, filtered and concentrated to obtain 1.57 g of a brown-yellow oily liquid as the target product ¹³C pyruvic acid (70.5% yield).

### "Two-step" preparation of labeled pyruvic acids

A magnetic stirring bar was added to a 100 mL well-dried, single-necked, round-bottomed flask, which is then filled with argon gas. Next, 50 mL of the isopropenyl Grignard reagent at a concentration of 1.0 mol/L (50 mmol total) was slowly added to the round-bottomed flask, followed by cooling the reaction vessel in an ethanol bath using a cryogenic circulating pump to cool the reaction vessel in an ethanol bath, and the round-bottomed flask was cooled to -78°C to allow rapid cooling of the isopropenyl Grignard reagent temperature.

A ¹³C-labeled carbon dioxide (1.12 L, 50 mmol) was slowly introduced to the condensed reaction vessel through a gas flow meter at a flow rate of 100 mL/min below the liquid level to fully react with the isopropenyl Grignard reagent. After all the ¹³C-labeled carbon dioxide was introduced to the reaction vessel, the reaction system was stirred with a magnetic stirring bar at -78°C for 1h to mix and react the ¹³C-labeled carbon dioxide with the isopropenyl Grignard reagent.

10 mL of hydrochloric acid solution with a concentration of 6N (6 mol/L) was added to the reaction system, and after the addition of hydrochloric acid was completed, the reaction system was heated to 0°C in a water bath. Subsequently, the reaction system was tested for the pH using a pH meter, and adjusted with dilute hydrochloric acid solution to pH 3 or less. It was to be noted that, in this embodiment, it was ensured that the acid used for the quenching reaction was the same as that used for pH adjustment; for example, when hydrochloric acid was used for performing the quenching reaction, dilute hydrochloric acid was also used for adjusting the pH; when sulfuric acid was used for performing the quenching reaction, dilute sulfuric acid was used for adjusting the pH.

In this embodiment, the isopropenyl Grignard reagent was cooled to -78°C and then continuously reacted with ¹³C-labeled carbon dioxide for 1 h. The acid was added into the reaction system and then heated to 0°C for a quenching reaction to free the carboxyl group in the carboxyl-magnesium salt complex.

In another embodiment, after the isopropenyl Grignard reagent was cooled to -60°C and added with the ¹³C-labeled carbon dioxide, the reaction system was heated to -30°C and reacted continuously for 1 h for a quenching reaction to free the carboxyl group in the carboxyl-magnesium salt complex.

In an embodiment, the isopropenyl Grignard reagent was cooled to -50°C, added with the ¹³C-labeled carbon dioxide and heated to -20°C to further accelerate the quenching reaction.

In an embodiment, the isopropenyl Grignard reagent was cooled to -30°C, added with the ¹³C-labeled carbon dioxide and then heated to -10°C to further accelerate the quenching reaction.

After the quenching reaction is finished, 300 mL of dichloromethane is added to the reaction vessel, the solution is transferred to a 500 mL separatory funnel, and after extracting and separating the liquid, the aqueous phase is extracted twice using dichloromethane, the organic phases are combined, dried with anhydrous sodium sulfate, filtered, and the organic solvent is evaporated, to obtain an oily product, which is used directly for the next step without purification. It should be noted that, in this embodiment, the drying agent in addition to anhydrous sodium sulfate, can also be used in other common drying agents, for example, anhydrous calcium chloride, anhydrous magnesium sulfate and so on.

In this embodiment, the volume of the reaction vessel used to carry out the addition reaction of the isopropenyl Grignard reagent with carbon dioxide is different from the volume of the reaction vessel used to carry out the ozonization reaction, and the volume of the reaction vessel used to carry out the ozonization reaction is larger in order to make it possible to introduce an excess of ozone into the reaction vessel when the ozonization reaction is carried out to make the reaction vessel form a supersaturated state, so as to facilitate the ozone reacting with the oily product sufficiently. Specifically, 50 mmol of the oily product obtained in the previous step was transferred to a 250 mL single-necked round-bottomed flask with a stirring magnet, 40 mL of methylene chloride and 40 mL of methanol were added to dissolve the oily product, and the reaction flask was then cooled to -78°C. An oxygen cylinder was connected to an ozone generator and ozone was slowly introduced into the reaction bottle below the liquid level, and after 15 min of reaction, the reaction liquid changed from light yellow to blue (the system turned blue indicating that there was ozone dissolved in the system, showing that the reaction was complete, and this embodiment judged the progress of the ozonization based on the color of the reaction liquid), and then the ozone generator was turned off and 5 min of oxygen was introduced in to exhaust the excess ozone. In another embodiment, the oily product can also be cooled to -50°C and then introduced into the ozone for the ozonization reaction, that is, the reaction temperature of the oily product with the ozone is increased to facilitate accelerating the process of the ozonization reaction of the oily product with the ozone. In the actual synthesis process, the temperature of the ozonization can be further adjusted as needed.

For the reduction reaction, 4.4 mL of dimethyl sulfide (60 mmol) was added to the reaction vessel, and stirring was continued at -78°C for 10 min, followed by raising to room temperature and stirring for 2 h to allow the product in step (S5) to be fully reduced in the presence of dimethyl sulfide.

Removal of impurities in the reduced product included: removing the organic phase of the reaction solution by rotary evaporator, followed by dissolving with 200 mL of dichloromethane, and then adjusting the solution to pH 9-11 with 10% aqueous sodium hydroxide solution, extracting and separating the liquid, and then washing the organic phase twice with deionized water and combining the aqueous phases, and then adjusting the pH to 1-5 with aqueous hydrochloric acid, then extracting the aqueous phases with dichloromethane for three times and combining the organic phases. The organic phase was finally dried with anhydrous sodium sulfate, and after filtration and concentration, 3.18 g of brown-yellow oily liquid was obtained, which was the product ¹³C pyruvic acid, and the yield of ¹³C pyruvic acid produced by the present method was assessed and calculated to be 71.5%.

In this embodiment, the process of preparing ¹⁴C-labeled carbon dioxide is used as an example to illustrate the synthesis method of ¹⁴C pyruvic acid.

Specifically, a negative-pressure fume hood was selected as the site for the synthesis of labeled pyruvic acid, and a stirring magnet was added to a well-dried, 50 mL capacity, two-necked round-bottomed flask filled with argon gas. Then Ba¹⁴CO₃ (986.5 mg, 5.0 mmol) was weighed and placed in a dry double-necked round-bottomed flask, and 10 mL of concentrated H₂SO₄ was measured and placed in a constant pressure dispensing funnel. At the same time, another fully dried single-necked round-bottomed flask with a capacity of 25 mL was taken and 1.0 mol/L the isopropenyl Grignard reagent (5.5 mL, 5.5 mmol) was added to the single-necked round-bottomed flask and filled with argon as protection, and the single-necked round-bottomed flask was subsequently cooled to -78°C (using a cryogenic recirculating pump to refrigerate the flask, and condensing with an ethanol bath).

The constant-pressure separatory funnel was connected to a double-necked round-bottomed flask, and the other mouth of the double-necked round-bottomed flask was connected to a one-necked round-bottomed flask containing Grignard reagent through a conduit, opened the constant-pressure separatory funnel, and slowly added concentrated sulfuric acid dropwise to produce a ¹⁴C-labeled carbon dioxide (112 mL, 5 mmol) through the gas conduit into the condensing single-necked round-bottomed flask below the liquid level, and the cooling temperature of the single-necked round-bottomed flask was adjusted to -50 ~ -20°C, the reaction was carried out for 1h, and then argon gas was introduced to the system to induce the complete reaction of ¹⁴C-labeled carbon dioxide with the isopropenyl Grignard reagent.

After complete reaction of ¹⁴C-labeled carbon dioxide with the isopropenyl Grignard reagent, 0.5 mL of 12 N (12 mol/L) hydrochloric acid solution was added to the reaction system, and the temperature of the reaction flask (i.e., a single-ported round-bottomed flask, and the same afterward) was heated to -50°C after the addition. Connected the oxygen cylinder to the ozone generator, and slowly introduced the ozone to the reaction bottle below the liquid level, after 15 min of reaction, the reaction liquid from light yellow to blue (the system becomes blue indicating that there is ozone dissolved in the system, showing that the reaction is complete), and then turn off the ozone generator, and introduce the oxygen for 5 min to drain away the excess ozone. Afterwards, 0.44 mL of dimethyl sulfide (6 mmol) was added to the reaction bottle, and then brought to room temperature and stirred for 2 h. Removed the organic phase of the reaction solution by rotary evaporator, then dissolved with 20 mL of dichloromethane, and then adjusted the pH value of the solution to 9-11 with 10% sodium hydroxide aqueous solution, extracted and separated, and then washed the organic phase with deionized water twice, combined the aqueous phase, and then adjusted the pH value of the reaction solution with hydrochloric acid aqueous solution to 1-5, and then extracted the aqueous phase with dichloromethane for 3 times, combined the organic phases, and then dried the organic phases with anhydrous sodium sulfate. Finally, filtered and concentrated to obtain the product ¹⁴C pyruvic acid.

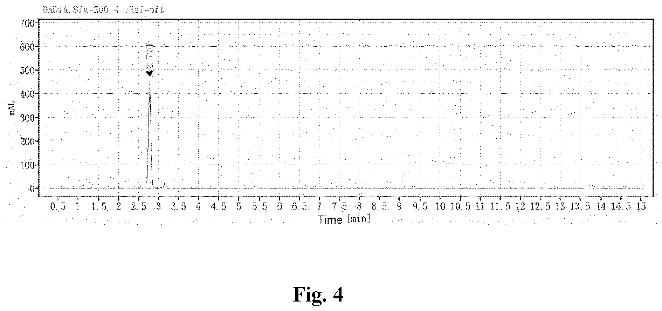

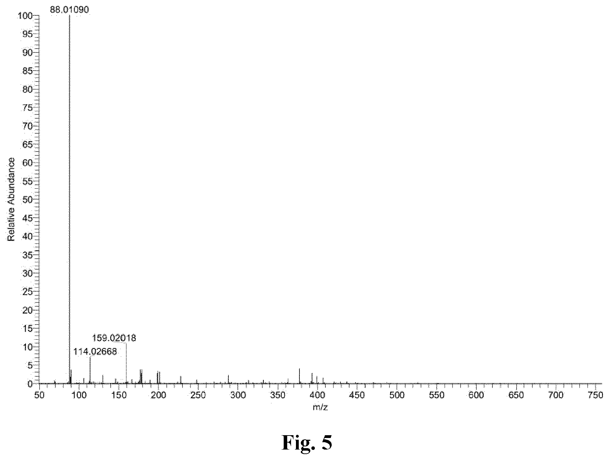

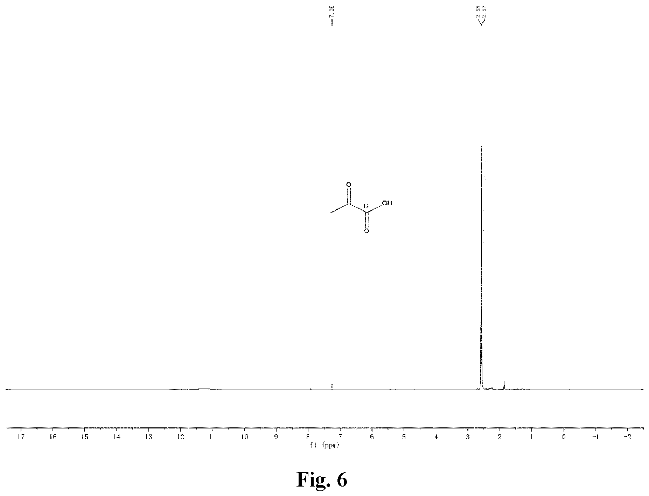

Through the synthesis method of this application, it can obtain labeled pyruvic acid with purity of more than 90%, this application has performed multiple spectrum tests on ¹³C pyruvate synthesized using the above method, please refer to Fig. 4, Fig. 4 illustrates the high performance liquid chromatography (HPLC) diagram of ¹³C pyruvate, using a column to perform the HPLC purity analysis, the parameters of the column chromatography were as follows: reversed-phase column 250x4.6mm, the mobile phase was acetonitrile: 0.02 mol/L potassium dihydrogen phosphate (30:70), pH adjusted to 3.0 with phosphoric acid, flow rate controlled at 1.0 ml/min, detection wavelength: 200 nm, injection volume: 20 µL, running time 15 min, and the final purity of ¹³C pyruvic acid measured was 92.91%. From the high-resolution mass spectrometry in Fig. 5 and the NMR hydrogen spectrum analysis in Fig. 6, it was seen that the abundance of ¹³C pyruvic acid could reach more than 98%, which made the synthesized ¹³C pyruvic acid's purity, abundance as well as yield able to satisfy the market needs. Fig. 6 illustrates the NMR hydrogen spectra of ¹³C pyruvic acid, according to which the type of chemical environment of the hydrogen atoms can be judged, and thus the labeling position of the ¹³C of this embodiment can be judged. Similarly, the purity, abundance, and yield of the ¹⁴C pyruvic acid synthesized by the method described above can meet the standards described above.

It is to be noted that in each of the above embodiments, the preparation process of ¹³C pyruvic acid and ¹⁴C pyruvic acid is exemplified only by the specific dosage of the material, and in the practical production, the experiments can also be expanded based on the ratios of the isopropenyl Grignard reagent, the carbon isotope-labeled carbon dioxide gas, and the rest of the various reagents in the above embodiments in order to meet the needs of industrial production, which will not be repeated herein.

The method for synthesizing a labeled pyruvic acid of this application uses an isopropenyl Grignard reagent and a carbon isotope-labeled carbon dioxide as the reaction starting materials, and there is no need to carry out the operations of "carboxylation protection" and "deprotection", compared with the traditional four-step method for synthesizing a labeled pyruvic acid, this application can synthesize a labeled pyruvic acid in only two steps, which reduces the number of steps in the synthesis reaction, improves the synthesis efficiency, reduces the amount and types of reagents used, and reduces the synthesis cost, and the synthesis reaction is free of other reagents, so that a labeled pyruvic acid can be obtained by extracting the final product, which reduces the difficulty of product extraction.

The various technical features of the above-described embodiments may be combined in any combination, and for the sake of conciseness of description, all possible combinations of the various technical features of the above-described embodiments have not been described, however, as long as there is no contradiction in the combinations of these technical features, all should be considered to be within the scope of the present specification as documented herein.

Described above are only several embodiments of the present disclosure, which are described in a more specific and detailed manner, but are not to be construed as a limitation on the scope of the disclosure. It should be pointed out that for those skilled in the art, several variations and improvements can be made without departing from the spirit of the present disclosure, all of which shall fall within the protection scope of the present disclosure defined by the appended claims.

## Claims

1. A method for synthesizing a labeled pyruvic acid, comprising:
(S1) adding an isopropenyl Grignard reagent to a reaction vessel followed by cooling to a preset temperature;
(S2) adding a carbon isotope-labeled carbon dioxide to the reaction vessel followed by reaction with the isopropenyl Grignard reagent for a preset time;
(S3) adding an acid to a reaction system obtained in step (S2) and adjusting the reaction system to a preset temperature to quench the reaction;
(S4) subjecting a quenched reaction system to ozonization to obtain an ozonized product;
(S5) reducing the ozonized product obtained in step (S4) with a reducing agent to obtain a reduced product; and
(S6) removing impurities from the reduced product to obtain the labeled pyruvic acid.

2. The method according to claim 1, **characterized in that** in step (S4), ozone is introduced to the quenched reaction system to continuously perform the ozonization for a preset time.

3. The method according to claim 1, **characterized in that** step (S4) comprises:
(S41) subjecting the quenched reaction system to extraction, and combining organic phases to obtain an oily product; and
(S42) adding a solvent to the oily product followed by cooling to a preset temperature and introduction of ozone to carry out the ozonization for a preset time.

4. The method according to claim 3, **characterized in that** in step (S3), the reaction system is added with a dilute hydrochloric acid to reach a pH of 3 or less.

5. The method according to claim 4, **characterized in that** before step (S42), the step (S4) further comprises:
(S411) transferring the oily product to another reaction vessel;
wherein in step (42), the solvent is a mixture of dichloromethane and methanol.

6. The method according to any one of claims 2-5, **characterized in that** the carbon isotope-labeled carbon dioxide is ¹³C-labeled carbon dioxide or ¹⁴C-labeled carbon dioxide.

7. The method according to claim 6, **characterized in that** in step (S1), before the isopropenyl Grignard reagent is added to the reaction vessel, a magnetic stirring bar is added to the reaction vessel, and argon is introduced to the reaction vessel; and the cooling is performed in an ethanol bath using a cryogenic recirculation pump.

8. The method according to claim 7, **characterized in that** the isopropenyl Grignard reagent is cooled to -30 to -78°C and then reacted with the carbon isotope-labeled carbon dioxide for 1 h; and
in step (S3), the preset temperature is -30 to 0°C.

9. The method according to claim 8, **characterized in that** the reducing agent in step (S5) is dimethyl sulfide or triphenylphosphine.

10. The method according to claim 9, **characterized in that** in step (S6), removal of impurities from the reduced product comprises:
(S61) removing an organic phase of the reduced product;
(S62) adjusting the reduced product to be alkaline followed by extraction and separation, and combining aqueous phases;
(S63) adjusting a combined aqueous phase to be acidic followed by extraction, and combining organic phases; and
(S64) subjecting a combined organic phase obtained in step (S63) to drying, filtration and concentration to obtain the labeled pyruvic acid.
